(19) Europäisches Patentamt
European Patent Office
Office européen des brevets

(11) Veröffentlichungsnummer: **0 306 870 A2**

# (12) EUROPÄISCHE PATENTANMELDUNG

(21) Anmeldenummer: **88114427.3**

(22) Anmeldetag: **03.09.88**

(51) Int. Cl.⁴: **C07K 13/00 , C12P 21/02 , C12N 15/00 , A61K 37/02 , //C07K15/26**

(30) Priorität: **10.09.87 DE 3730331**

(43) Veröffentlichungstag der Anmeldung: **15.03.89 Patentblatt 89/11**

(84) Benannte Vertragsstaaten: **AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Schollmeier, Klaus, Dr.**
**Landstrasse 80 a**
**D-6944 Hemsbach(DE)**
Erfinder: **Kreimeyer, Andreas, Dr.**
**Josef-Schmidt-Strasse 5**
**D-6720 Speyer(DE)**
Erfinder: **Daum, Lothar, Dr.**
**Reiherstrasse 25**
**D-6701 Otterstadt(DE)**

## (54) Gamma-Interferon-Derivate, Herstellungsverfahren, Vektoren dafür und Arzneimittel daraus.

(57) Es werden Peptide beschrieben, die sich von Interferon-γ durch Abspaltung von 7 bis 11 Aminosäuren am C-terminalen Ende unterscheiden. Weiter wird deren gentechnologische Herstellung angegeben. Die neuen Peptide lassen sich zur Herstellung von Arzneimitteln verwenden.

EP 0 306 870 A2

Xerox Copy Centre

## Neue Polypeptide, Verfahren zur Herstellung, die Vektoren dafür und Arzneimittel daraus

Die Erfindung betrifft neue Polypeptide mit Interferon-γ-Aktivität oder Interferon-γ-ähnlicher Aktivität, Verfahren zu deren Herstellung, die Vektoren dafür und Arzneimittel, die diese Polypeptide enthalten.

Das Interferon-γ (früher Immuninterferon oder Typ II-Interferon bezeichnet) wurde im Jahre 1965 von F. Wheelock entdeckt (Science, 149, 310, 1965), der zeigte, daß Interferon-γ bestimmte Zellen vor einer Virusinfektion schützen kann.

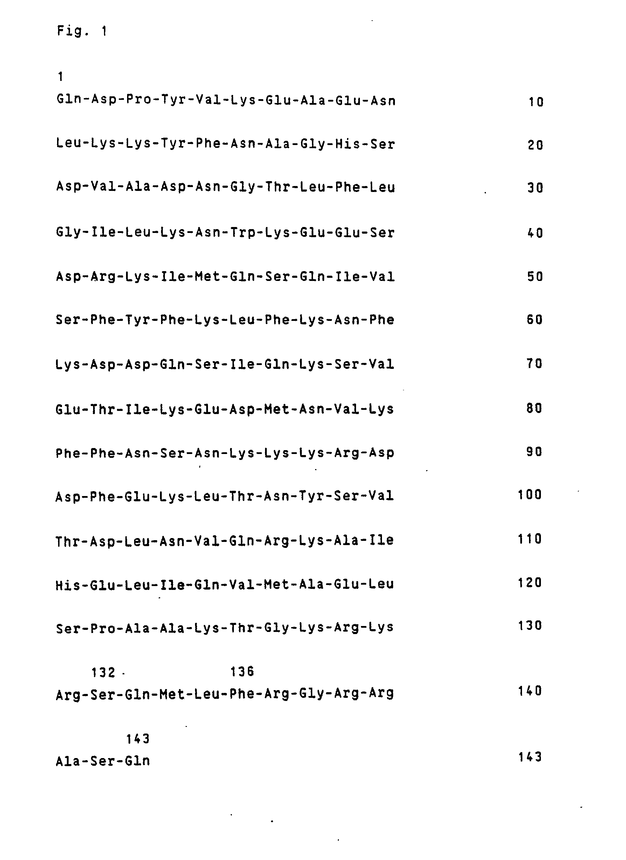

Die ursprünglich veröffentlichte Aminosäuresequenz von Humaninterferon-γ weist 146 Aminosäuren auf (Nucl. Acid. Res. 10, 2487, 1982). Nach einer späteren Veröffentlichung (J. Biol. Chem., 259, 6790, 1984) fehlen jedoch dem "reifen" Interferon-γ die ersten drei Aminosäuren aus der ursprünglich publizierten Sequenz. Im folgenden wird daher die Aminosäuresequenz von der Aminosäure Nr. 1 (Glutamin) bis zur Aminosäure Nr. 143 (Glutamin) als reifes, humanes Interferon-γ bezeichnet. Die Aminosäuresequenz dieses Moleküls ist in Fig. 1 wiedergegeben.

Menschliches Interferon-γ (W.E. Stewart, II, The Interferon Sys., Springer-Verlag, 2nd ed. 1981) ist ein Polypeptid, das von Natur aus glykosyliert ist (Nature 295, 503, 1982). Das Glycoprotein besitzt ein Molekulargewicht von etwa 63.000 bis 73.000 da (J. Biol. Chem., 258, 9706, 1983) und liegt in seiner funktionellen Form wahrscheinlich als Dimer vor. Die Glykosylierung des Interferon-γ ist für seine Wirkung nicht erforderlich. So reduziert die Behandlung von Interferon-γ mit Glykosidase nicht dessen antivirale Aktivität in humanen Fibroblasten-Zellkulturen (J. Biol. Chem., 258, 8010, 1983). Aus der EP-OS 146 944 ist die gentechnische Herstellung von Derivaten des Interferon-γ bekannt, bei denen in Position 6 Lysin durch Glutamin ersetzt ist und zusätzlich weitere Modifikationen durchgeführt sind. Unter diesen Modifikationen sind auch N-terminale Verkürzungen um 1 bzw. 4 Aminosäuren, die durch Bal 31-Verdauung der DNA und Expression gewonnen wurden. Diese Derivate des Interferon-γ sollen eine dem natürlichen Interferon-γ vergleichbare oder sogar erhöhte Aktivität besitzen.

Es wurde auch schon vorgeschlagen (DE-OS 3 414 831), Derivate des Interferon-γ herzustellen, bei denen die erste Aminosäure der natürlichen Sequenz und/oder die nach der Aminosäure 124 (Alanin) folgenden Amino
säuren entfallen, ohne daß die biologische Aktivität des Interferon-γ nennenswert beeinträchtigt wäre.

Es wurde nun gefunden, daß bei einer Abspaltung der C-terminalen 7, 8, 9, 10 oder 11 Aminosäuren, Peptide erhalten werden, die eine signifikant höhere biologische Aktivität besitzen. Gegenstand der Erfindung sind Polypeptide der in Anspruch 1 angegebenen Formel. Weiterhin betrifft die Erfindung die Herstellung dieser Polypeptide in Bakterien sowie dazu verwendeten Vektoren bzw. DNA-Sequenzen.

Bei der gentechnologischen Herstellung in E.coli können diese neuen Polypeptide auch an ihrem N-terminalen Ende methioniert oder mit einer anderen Peptidsequenz versehen sein. Diese Peptidsequenz kann eine für die Sekretion verantwortliche Signalsequenz oder eine spezifische Erkennungssequenz für z.B. Fibrin oder andere Zelloberflächenstrukturen beinhalten.

Die neuen Peptide lassen sich herstellen, indem man

a. das Interferon-γ-Gen in einen M 13-Vektor insertiert,

b. daraus eine einzelsträngige DNA isoliert und diese in einem Mutagenese-Ansatz mit einem Oligonucleotid hybridisiert und dadurch einen gezielten Basenaustausch bewirkt, der in der kodierenden Region des Interferon-γ-Gens ein Stop-Codon kreiert,

c. dieses modifizierte Interferon-γ-Gen isoliert,

d. dieses Gen in einen geeigneten E-coli-Expressions-Vektor insertiert und

e. mit Hilfe dieses Expressions-Vektors das Polypeptid exprimiert und isoliert.

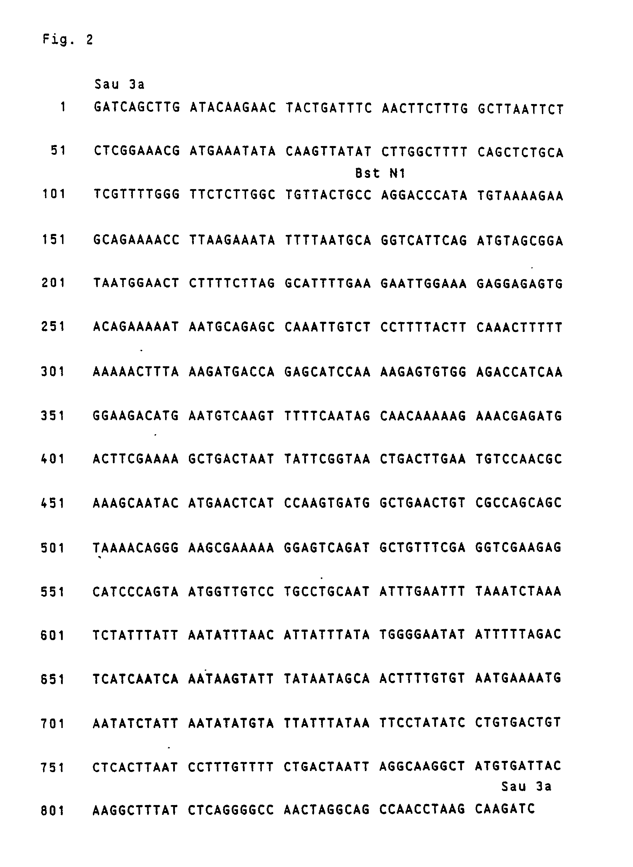

Bei der Herstellung der erfindungsgemäßen Polypeptide ist es besonders vorteilhaft, eine durch gerichtete Mutagenese des Interferon-γ-Gens erhaltene DNA zu verwenden. Als Ausgangsmaterial für dieses Verfahren diente die Interferon-γ-cDNA (Abb. 2), die für die in Abb. 1 dargestellte Interferon-γ-Aminosäuresequenz von Pos. 1 bis Pos. 143 kodiert.

Aufgrund der Degeneration des gentischen Codes ist es aber auch möglich, andere DNA-Sequenzen, z.B. chemisch synthetisierte Gene mit unterschiedlicher DNA-Sequenz für die Expression der neuen Polypeptide zu benutzen. Die erfindungsgemäß erhaltenen Polypeptide besitzen eine Interferon-γ oder Interferon-γ-ähnliche Aktivität und können bei Tumorerkrankungen, bei Immunkrankheiten oder bei Entzündungskrankheiten wie Rheuma oder Polyarthritis eingesetzt werden. Sie werden dabei in Mengen von 5 bis 500 µg pro ml Körperoberfläche und Tag eingesetzt.

Gegenstand der Erfindung sind daher auch Arzneimittel, die mindestens eines der neuen Peptide enthalten, gegebenenfalls in einem pharmazeutisch verträglichen Träger oder Bindemittel, insbesondere Human-Serumalbumin. Die Arzneimittel können auch Kombinationen der neuen Proteine mit schon bekannten Lymphokinen (z.B. TNF, Lymphotoxin) oder Lymphokin-Mutanten (EP-OS 250.000, insbesondere Δ24-Lymphotoxin) enthalten. Weitere Ausgestaltungen der Erfindungen sind in den folgenden Beispielen näher beschrieben. Das als Vergleichssubstanz dienende Interferon-γ wurde in bekannter Weise in E-coli cloniert, exprimiert und wie beschrieben aufgereinigt (Methods in Enzymology; 119; 366-383; 1986).

Beispiel 1

Herstellung eines Hybridplasmids, das das Gen für Interferon-γ als Vergleichssubstanz enthält.

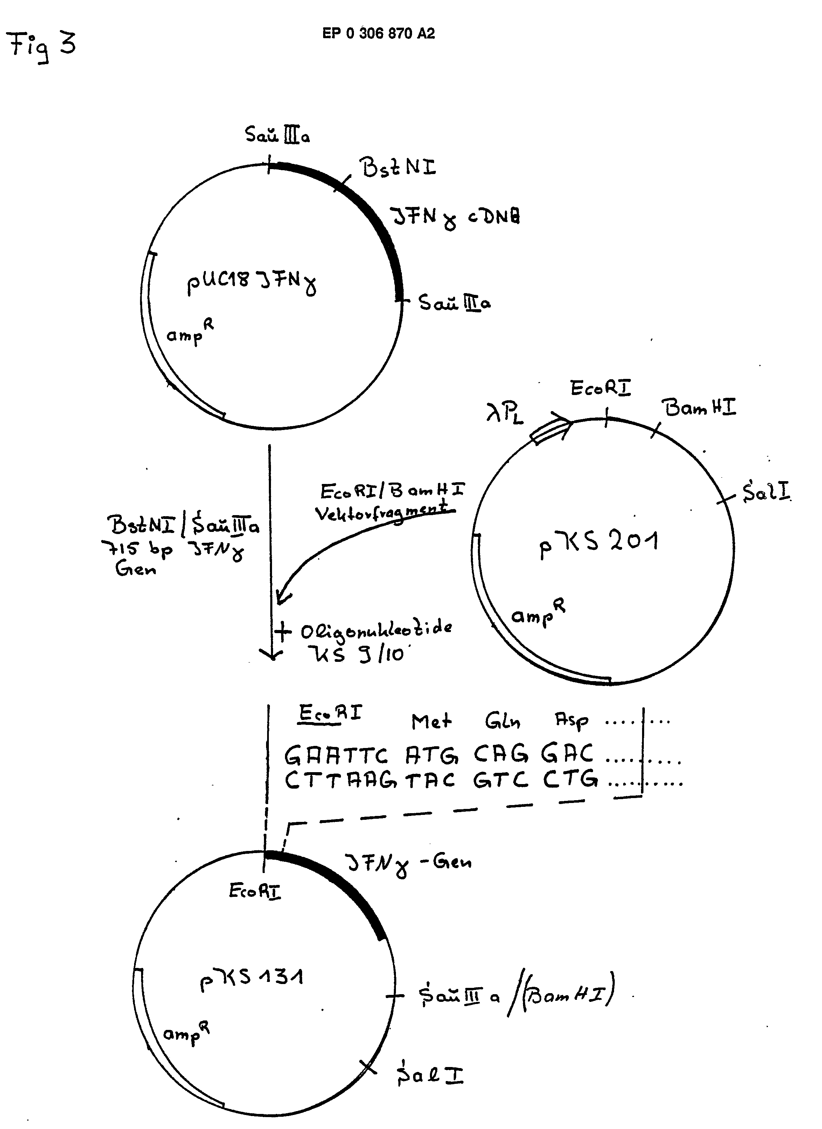

Ausgangspunkt war das Plasmid pUC 18 IFN-γ. Es entstand durch die Insertion der mit Sau IIIa geschnittenen Interferon-γ cDNA (Fig. 2) in die Bam HI Erkennungsstelle von pUC 18. Das Plasmid pUC 18 IFN-γ wurde dann in bekannter Weise mit den Restriktionsendonucleasen Bst NI/Sau IIIa nach den Angaben des Herstellers geschnitten (Fig. 3), die Bruchstücke auf einem 5 %igen Polyacrylamidgel durch Elektrophorese aufgetrennt und durch Anfärben mit Ethidiumbromid sichtbar gemacht. Das benötigte Interferon-γ-Gen (Bst NI/Sau IIIa-715 bp) wurde anschließend aus dem Acrylamidgel ausgeschnitten und elektrophoretisch von der Matrix abgetrennt (Bio-Trap® von Firma Schleicher und Schüll). 0,1 pMol dieses Fragments wurden dann mit 0,1 pMol Vektor (pKS 201 Eco RI/Bam HI) und den synthetischen Oligonukleotiden KS 9/10 über Nacht bei 15° C ligiert.

```
KS 9    5'  AATTCATGC    3'
KS 10   3'      GTACGT   5'
```

Man erhielt das Hybridplasmid pKS 131 gemäß Fig. 3. Dieses Hybridplasmid wurde an seiner Eco RI Schnittstelle mit synthetisch hergestellten Translationssignalsequenzen versehen und das IFN-γ-Gen in den entsprechenden E.coli-Stämmen mit dem temperatursensitiven γ-Repressor (cI 857) bei 42° C exprimiert.

Das so hergestellte IFN-γ mit den in Fig. 1 angegebenen Aminosäuresequenzen (plus dem N-terminalen Methionin) diente als Vergleichssubstanz.

Beispiel 2

Proteinchemische Reinigung der IFN-γ-Referenzsubstanz.

1. Schritt: Gewinnung und Reinigung der IFN-γ-Einschlußkörper

Identisch mit Beispiel 4, 1. Schritt.

2. Schritt: Renaturierung

Identisch mit Beispiel 4, 2. Schritt.

3. Schritt: Verdünnung des Renaturates

Das Renaturat wird 1 : 1,7 mit 20 mM Ammoniumacetat pH 7,5 verdünnt und anschließend 30 Minuten bei 8000 g zentrifugiert.

4. Schritt: Ionenaustauschchromatographie

Wie Beispiel 4, 4. Schritt jedoch mit veränderten Puffern
Äquilibrierungspuffer:
0,4 M Argininhydrochlorid/3 % Saccharose/20 mM Ammoniumacetat pH 7,5
Elutionspuffer:
0,4 M Argininhydrochlorid/3 % Saccharose/20 mM $NaH_2PO_4$ pH 7,5 mit steigenden NaCl-Gehalt (0 bis 0,15 M)
Als Interferon-γ eluiert unter den angegebenen Bedingungen zwischen 0,07 und 0,15 M NaCl.

5. Schritt: Hydrophobe Chromatographie

Identisch mit Beispiel 4, 5. Schritt.

Beispiel 3

Herstellung der neuen Polypeptide

Das Plasmid pkS 131 wurde mit den Restriktionsnukleasen Eco RI/Sal I geschnitten und das DNA-Fragment mit dem IFN-γ-Gen in den mit Eco RI und Sal I geschnittenen M 13-Vektor mp 11 insertiert. Dieses neue Plasmid wurde in E.coli BMH 71-18 transformiert und dann der daraus resultierende M 13-Einzelstrang isoliert. Neben diesem Einzelstrang wurde noch das Eco RI/ Sal I Vektorfragment von M 13 mp 19 isoliert und zusammen mit dem Einzelstrang in den "gapped duplex"-Ansatz eingesetzt.

Das Vorgehen bei der M 13 Mutagenese ist in Nucleic Acids Research 12, 9441, 1984 und Molecular Cloning Laboratory Manual, Cold Spring Harbor, 1982 beschrieben. Alle Arbeiten wurden mit dem M 13-site directed mutagenese kit von Boehringer, Mannheim (Nr. 10 27 492) durchgeführt.

Für die Einführung der Mutationen wurden folgende Oligonukleotide benutzt.

| | |
|---|---|
| KS 6 | GGAGT**T**AGATGCTGTTTCG |
| KS 7 | GGAGTCAG**TA**GCTGTTTCG |
| KS 8 | GTCAGATG**TA**GTTTCGAGG |
| KS 9 | GATGCTGT**AA**CGAGGTCG |
| KS 10 | GATGCTGTTT**T**GAGGTCG |

Mit diesen Oligonukleotiden wurden Punktmutationen in die DNA-Sequenz für IFN-γ eingebracht. Diese Punktmutationen bewirken vorzeitige Stopps im Leseraster und somit entstehen Gene für C-terminal verkürzte IFN-γ-Moleküle.

Nach der Mutagenese und der Identifizierung der richtigen Klone mittels Sanger-Sequenzierung wurden die entsprechenden neuen Gene mit Eco RI/Sal I aus dem M 13-Vektor wieder herausgeschnitten und in den Eco RI/Sal I geschnittenen Vektor pkS 201 insertiert (siehe auch Fig. 3). Wie schon in Beispiel 1 beschrieben, wurden in die einzelnen Eco RI-Schnittstellen Signalsequenzen für die Expression insertiert und die neuen Peptide exprimiert, gereinigt und charakterisiert. Je nach benutzem Oligonukleotid bei der Mutagenese entstanden so:

KS6 : AS 1-132 = delta 11 IFN-γ
KS7 : AS 1-133 = delta 10 IFN-γ
KS8 : AS 1-134 = delta 9 IFN-γ
KS9 : AS 1-135 = delta 8 IFN-γ
KS10: AS 1-136 = delta 7 IFN-γ

Die neuen Polypeptide enthielten noch jeweils ein Methionin am Aminoterminus.

Beispiel 4

Proteinchemische Reinigung der neuen Polypeptide

Zur proteinchemischen Reinigung aller IFN-γ-Muteine wurden jeweils die folgenden Aufarbeitungsschritte durchgeführt:

1. Schritt: Gewinnung und Reinigung der IFN-γ-Mutein-Einschlußkörper

Zur Gewinnung und Reinigung der Interferon-γ-Mutein-Einschlußkörper aus dem jeweiligen Produktionsstamm wurden die E.coli-Zellen aus der Fermentationsbrühe durch Zentrifugation (6000 bis 8000 g, 30 min.) gewonnen und dann mit Hilfe einer Ultraschallsonde (5 × 2 min, 150 W, Microtip) aufgeschlossen. Die Einschlußkörper wurden 3mal mit 20 mM Phosphatpuffer pH 7,5, 20 mM EDTA (5 ml/g E.coli) gewaschen und in einem Guanidin-Hydrochlorid-haltigen Puffer (3M Guanidin-Hydrochlorid/0,4M Arginin/20 mM Ammoniumacetat pH 7,5/2,5 ml pro g E.coli) 48 h bei 4° C gerührt, wobei sich die Einschlußkörper lösten. Kontaminierende DNA wurde durch $MnCl_2$-Fällung entfernt (40 mM $MnCl_2$-Endkonzentration). Die Interferon-γ-Mutein-Ausbeute bei diesem Verfahren betrug 60 bis 70 %, die proteinchemische Reinheit ca. 50 %.

2. Schritt: Renaturierung

In Guanidin-Hydrochlorid gelöstes, grobgereinigtes Interferon-γ-Mutein wurde renaturiert, indem das durch Schritt 1 gewonnene Material unter stetem Rühren über einen Zeitraum von 1 h zu Renaturierungspuffer (0,4M Arginin/10 % Saccharose/20 mM Ammoniumacetat pH 7,5) gepumpt wurde. Es wurde so viel Renaturierungspuffer vorgelegt, daß eine Interferon-γ-Mutein-Endkonzentration von 0,4 bis 0,8 mg/ml entstand. Der Ansatz wurde nach Erreichen der Endkonzentration über Nacht bei 4° C weitergerührt.

3. Schritt: Verdünnung des Renaturates

Das Renaturat wurde 1:2 mit Wasser verdünnt und anschließend 30 min bei 8000 g zentrifugiert.

4. Schritt: Ionenaustauschchromatographie

Der verdünnte, zentrifugierte Renaturierungsansatz wurde über den Anionenaustauscher Q-Sepharose® auf eine S-Sepharosesäule gepumpt. Auf dem Anionenaustauscher erfolgte eine Abreicherung von DNA und Lipid-haltigen Fragmenten, während das Interferon-γ hindurchlief und an den Kationenaustauscher S-Sepharose® band. Die Säulenkombination wurde mit Äquilibrierungspuffer (20 mM Ammoniumacetat, 0,25M Arginin-HCl, 3 % Saccharose, pH 7,5) gewaschen. Die S-Sepharose®-Säule wurde nun abgekoppelt und ohne Q-Sepharose® wie folgt weiterentwickelt:

- Waschen mit 3 Säulenvolumina Äquilibrierungspuffer
- Eluieren des IFN-γ mit 20 mM Ammonumacetat/3 % Saccharose pH 7,5 mit steigendem Arginin-HCl-Gehalt (0,25 bis 0,4 M)

5. Schritt: Hydrophobe Chromatographie

Die von der S-Sepharose®-Säule eluierte Fraktion mit der gesuchten Aktivität wurde durch Zugabe von festem Ammoniumsulfat auf eine Endkonzentration von 0,3M eingestellt und auf eine mit 0,3M Ammoniumsulfat/0,2M Arginin/3 % Saccharose/5 mM Phosphatpuffer pH 7,0 äquilibrierte Phenyl-Sepharose® der Firma Pharmacia gegeben. Unter den gewählten Bedingungen lief das jeweils neue Polypeptid durch die Säule hindurch, während die E.coli Proteine zum Großteil an die Matrix banden. Das Protein-haltige Eluat wurde über eine 10kDa-Membran der Firma Amicon 10-20fach konzentriert.

Die so erhaltenen Konzentrate enthielten die neuen Polypeptide in Mengen von 1,0 bis 1,6 mg/ml.

Beispiel 5

Bestimmung der antiviralen Aktivität

Hierzu wurden 100 µl Kulturmedium mit 2 x $10^4$ HEp-2-Zellen in die Vertiefungen einer 96-Loch-Flachbodenplatte gegeben und über Nacht bei 37° C und 5 % (V/V) Kohlendioxid inkubiert. Am nächsten Tag wurden 100 µl der IFN-γ-haltigen Probelösung, eingestellt auf 10 ng Protein/ml, zu den konfluenten Zellkulturen zugegeben und seriell 2fach titriert.

Ebenso wurden 100 µl einer international definierten IFN-γ-Standardlösung (Gg 23-901-530, NIH, Bethesda, Maryland, USA) mitgeführt.

Auf der Kulturplatte wurden zudem eine Zellkontrolle (= unbehandelte, nicht mit Virus infizierte Zellen) und eine Viruskontrolle (= unbehandelte, mit Virus infizierte Zellen) mitangelegt. Nach einer weiteren Inkubatioszeit von 24 h bei 37° C und 5 % (V/V) $CO_2$ wurden die Kulturen mit 50 µl einer VSV-Suspension in Kulturmedium infiziert und bei 37° C und 5 % (V/V) $CO_2$ inkubiert. Nach zwei Tagen war die virusbedingte Zellzerstörung (CPE) in den ungeschützten Kulturen (= Viruskontrolle) abgelaufen. Der Prozentsatz geschützter Zellen in den mit der Testverbindung behandelten und anschließend mit VSV infizierten Kulturen wurde mittels Kristallviolettfärbung bestimmt.

Danach wurde bezogen auf 0 und 100 % Schutz der 50 % Schutz definiert und der Kehrwert der Probenverdünnung, die 50 % Schutz führt, als Konzentration der antiviralen Aktivität der untersuchten Probe in Einheiten/mg angegeben. Die Einheiten des internationalen Standards wurden analog bestimmt. Fand sich eine Abweichung von dem für den Standard festgesetzten Wert, wurde der entsprechende Korrekturfaktor ermittelt und die Meßwerte korrigiert.

Wie die Tabelle zeigt, besitzen die neuen Polypeptide eine etwa 6fach höhere antivirale Aktivität als IFN-γ.

Tabelle

| Antivirale Aktivitäten der neuen Polypeptide | | |
|---|---|---|
| Polypeptid | antivirale Aktivität (Internationale Einheiten/mg) | relative Aktivität |
| IFN-γ | $9,4 \times 10^6$ | 1 |
| Δ 7 IFN-γ | $52,5 \times 10^6$ | 5,6 |
| Δ 10 IFN-γ | $57,4 \times 10^6$ | 6,1 |
| Δ 11 IFN-γ | $58,4 \times 10^6$ | 6,2 |

## Ansprüche

1. Polypeptide der Formel

X-Asp-Pro-Tyr-Val-Lys-Glu-Ala-Glu-Asn
Leu-Lys-Lys-Tyr-Phe-Asn-Ala-Gly-His-Ser
Asp-Val-Ala-Asp-Asn-Gly-Thr-Leu-Phe-Leu
Gly-Ile-Leu-Lys-Asn-Trp-Lys-Glu-Glu-Ser
Asp-Arg-Lys-Ile-Met-Gln-Ser-Gln-Ile-Val

Ser-Phe-Tyr-Phe-Lys-Leu-Phe-Lys-Asn-Phe
Lys-Asp-Asp-Gln-Ser-Ile-Gln-Lys-Ser-Val
Glu-Thr-Ile-Lys-Glu-Asp-Met-Asn-Val-Lys
Phe-Phe-Asn-Ser-Asn-Lys-Lys-Lys-Arg-Asp
Asp-Phe-Glu-Lys-Leu-Thr-Asn-Tyr-Ser-Val
Thr-Asp-Leu-Asn-Val-Gln-Arg-Lys-Ala-Ile
His-Glu-Leu-Ile-Gln-Val-Met-Ala-Glu-Leu
Ser-Pro-Ala-Ala-Lys-Thr-Gly-Lys-Arg-Lys
Arg-Y

worin
X für Met-Gln oder Gln und
Y für
Ser,
Ser-Gln,
Ser-Gln-Met,
Ser-Gln-Met-Leu oder
Ser-Gln-Met-Leu-Phe,

stehen, sowie deren Allelvariationen.

2. Polypeptide nach Anspruch 1, enthaltend zusätzlich eine immunogen wirkende Peptidsequenz am N-terminalen Ende.

3. Verfahren zur Herstellung der Polypeptide nach Anspruch 1 und 2, dadurch gekennzeichnet, daß man

a) das Interferon-γ-Gen in einen M 13-Vektor insertiert,

b) daraus eine einzelsträngigè DNA isoliert und diese in einem Mutagenese-Ansatz mit einem Oligonucleotid hybridisiert und dadurch einen gezielten Basenaustausch bewirkt, der in der kodierenden Region des Interferon-γ-Gens ein Stop-Codon kreiert,

c) dieses modifizierte Interferon-γ-Gen isoliert,

d) dieses Gen in einen geeigneten E-coli-Expressionsvektor insertiert und

e) mit Hilfe dieses Expressions-Vektors das Polypeptid exprimiert und isoliert.

4. Vektoren, die Gensequenzen enthalten, die für die Polypeptide gemäß den Ansprüchen 1 und 3 kodieren.

5. Vektoren nach Anspruch 5 mit den Gensequenzen der Formel

```
X-GACCCATA TGTAAAAGAA GCAGAAAACC TTAAGAAATA TTTTAATGCA
GGTCATTCAG ATGTAGCGGA TAATGGAACT CTTTTCTTAG GCATTTTGAA
GAATTGGAAA GAGGAGAGTG ACAGAAAAAT AATGCAGAGC CAAATTGTCT
CCTTTTACTT CAAACTTTTT AAAAACTTTA AAGATGACCA GAGCATCCAA
AAGAGTGTGG AGACCATCAA GGAAGACATG AATGTCAAGT TTTTCAATAG
CAACAAAAAG AAACGAGATG ACTTCGAAAA GCTGACTAAT TATTCGGTAA
CTGACTTGAA TGTCCAACGC AAAGCAATAC ATGAACTCAT CCAAGTGATG
GCTGAACTGT CGCCAGCAGC TAAAACAGGG AAGCGAAAAA GG-Y
```

wobei
X für ATG oder ATG CAG

und Y für
AGT TAG
AGT CAG TAG
AGT CAG ATG TAG
AGT CAG ATG CTG TAA
AGT CAG ATG CTG TTT TGA steht.

6. DNA-Sequenzen codierend für ein Polypeptid gemäß Anspruch 1.

7. DNA-Sequenzen codierend für ein Polypeptid gemäß Anspruch 2.

8. Arzneimittel, enthaltend ein Polypeptid gemäß einem der Ansprüche 1 oder 2.

9. Arzneimittel gemäß Anspruch 8, enthaltend ein Polypeptid gemäß Anspruch 1 oder 2 und ein Lymphokin.

10. Arzneimittel gemäß Anspruch 9, enthaltend als Lymphotein TNF-α, TNF-β oder deren Muteine.

Patentanspruch für folgenden Vertragsstaat: ES;

Verfahren zur Herstellung der Polypeptide der Formel

X-Asp-Pro-Tyr-Val-Lys-Glu-Ala-Glu-Asn
Leu-Lys-Lys-Tyr-Phe-Asn-Ala-Gly-His-Ser
Asp-Val-Ala-Asp-Asn-Gly-Thr-Leu-Phe-Leu
Gly-Ile-Leu-Lys-Asn-Trp-Lys-Glu-Glu-Ser
Asp-Arg-Lys-Ile-Met-Gln-Ser-Gln-Ile-Val
Ser-Phe-Tyr-Phe-Lys-Leu-Phe-Lys-Asn-Phe
Lys-Asp-Asp-Gln-Ser-Ile-Gln-Lys-Ser-Val
Glu-Thr-Ile-Lys-Glu-Asp-Met-Asn-Val-Lys
Phe-Phe-Asn-Ser-Asn-Lys-Lys-Lys-Arg-Asp
Asp-Phe-Glu-Lys-Leu-Thr-Asn-Tyr-Ser-Val
Thr-Asp-Leu-Asn-Val-Gln-Arg-Lys-Ala-Ile
His-Glu-Leu-Ile-Gln-Val-Met-Ala-Glu-Leu
Ser-Pro-Ala-Ala-Lys-Thr-Gly-Lys-Arg-Lys
Arg-Y

worin
X für Met-Gln oder Gln und
Y für Ser,
Ser-Gln,
Ser-Gln-Met,
Ser-Gln-Met-Leu oder
Ser-Gln-Met-Leu-Phe,

stehen, sowie deren Allelvariationen.

a) das Interferon-γ-Gen in einen M 13-Vektor insertiert,

b) daraus eine einzelsträngige DNA isoliert und diese in einem Mutagenese-Ansatz mit einem Oligonucleotid hybridisiert und dadurch einen gezielten Basenaustausch bewirkt, der in der kodierenden Region des Interferon-γ-Gens ein Stop-Codon kreiert,

c) dieses modifizierte Interferon-γ-Gen isoliert,

d) dieses Gen in einen geeigneten E-coli-Expressionsvektor insertiert und

e) mit Hilfe dieses Expressions-Vektors das Polypeptid exprimiert und isoliert.

BASF Aktiengesellschaft 870504

Fig. 1

| | |
|---|---|
| 1 | |
| Gln-Asp-Pro-Tyr-Val-Lys-Glu-Ala-Glu-Asn | 10 |
| Leu-Lys-Lys-Tyr-Phe-Asn-Ala-Gly-His-Ser | 20 |
| Asp-Val-Ala-Asp-Asn-Gly-Thr-Leu-Phe-Leu | 30 |
| Gly-Ile-Leu-Lys-Asn-Trp-Lys-Glu-Glu-Ser | 40 |
| Asp-Arg-Lys-Ile-Met-Gln-Ser-Gln-Ile-Val | 50 |
| Ser-Phe-Tyr-Phe-Lys-Leu-Phe-Lys-Asn-Phe | 60 |
| Lys-Asp-Asp-Gln-Ser-Ile-Gln-Lys-Ser-Val | 70 |
| Glu-Thr-Ile-Lys-Glu-Asp-Met-Asn-Val-Lys | 80 |
| Phe-Phe-Asn-Ser-Asn-Lys-Lys-Lys-Arg-Asp | 90 |
| Asp-Phe-Glu-Lys-Leu-Thr-Asn-Tyr-Ser-Val | 100 |
| Thr-Asp-Leu-Asn-Val-Gln-Arg-Lys-Ala-Ile | 110 |
| His-Glu-Leu-Ile-Gln-Val-Met-Ala-Glu-Leu | 120 |
| Ser-Pro-Ala-Ala-Lys-Thr-Gly-Lys-Arg-Lys | 130 |
| 132 136 | |
| Arg-Ser-Gln-Met-Leu-Phe-Arg-Gly-Arg-Arg | 140 |
| 143 | |
| Ala-Ser-Gln | 143 |

870504

BASF Aktiengesellschaft

Fig. 2

```
        Sau 3a
  1     GATCAGCTTG ATACAAGAAC TACTGATTTC AACTTCTTTG GCTTAATTCT

 51     CTCGGAAACG ATGAAATATA CAAGTTATAT CTTGGCTTTT CAGCTCTGCA
                                        Bst N1
101     TCGTTTTGGG TTCTCTTGGC TGTTACTGCC AGGACCCATA TGTAAAAGAA

151     GCAGAAAACC TTAAGAAATA TTTTAATGCA GGTCATTCAG ATGTAGCGGA

201     TAATGGAACT CTTTTCTTAG GCATTTTGAA GAATTGGAAA GAGGAGAGTG

251     ACAGAAAAAT AATGCAGAGC CAAATTGTCT CCTTTTACTT CAAACTTTTT

301     AAAAACTTTA AAGATGACCA GAGCATCCAA AAGAGTGTGG AGACCATCAA

351     GGAAGACATG AATGTCAAGT TTTTCAATAG CAACAAAAAG AAACGAGATG

401     ACTTCGAAAA GCTGACTAAT TATTCGGTAA CTGACTTGAA TGTCCAACGC

451     AAAGCAATAC ATGAACTCAT CCAAGTGATG GCTGAACTGT CGCCAGCAGC

501     TAAAACAGGG AAGCGAAAAA GGAGTCAGAT GCTGTTTCGA GGTCGAAGAG

551     CATCCCAGTA ATGGTTGTCC TGCCTGCAAT ATTTGAATTT TAAATCTAAA

601     TCTATTTATT AATATTTAAC ATTATTTATA TGGGGAATAT ATTTTTAGAC

651     TCATCAATCA AATAAGTATT TATAATAGCA ACTTTTGTGT AATGAAAATG

701     AATATCTATT AATATATGTA TTATTTATAA TTCCTATATC CTGTGACTGT

751     CTCACTTAAT CCTTTGTTTT CTGACTAATT AGGCAAGGCT ATGTGATTAC
                                                     Sau 3a
801     AAGGCTTTAT CTCAGGGGCC AACTAGGCAG CCAACCTAAG CAAGATC
```

Fig 3

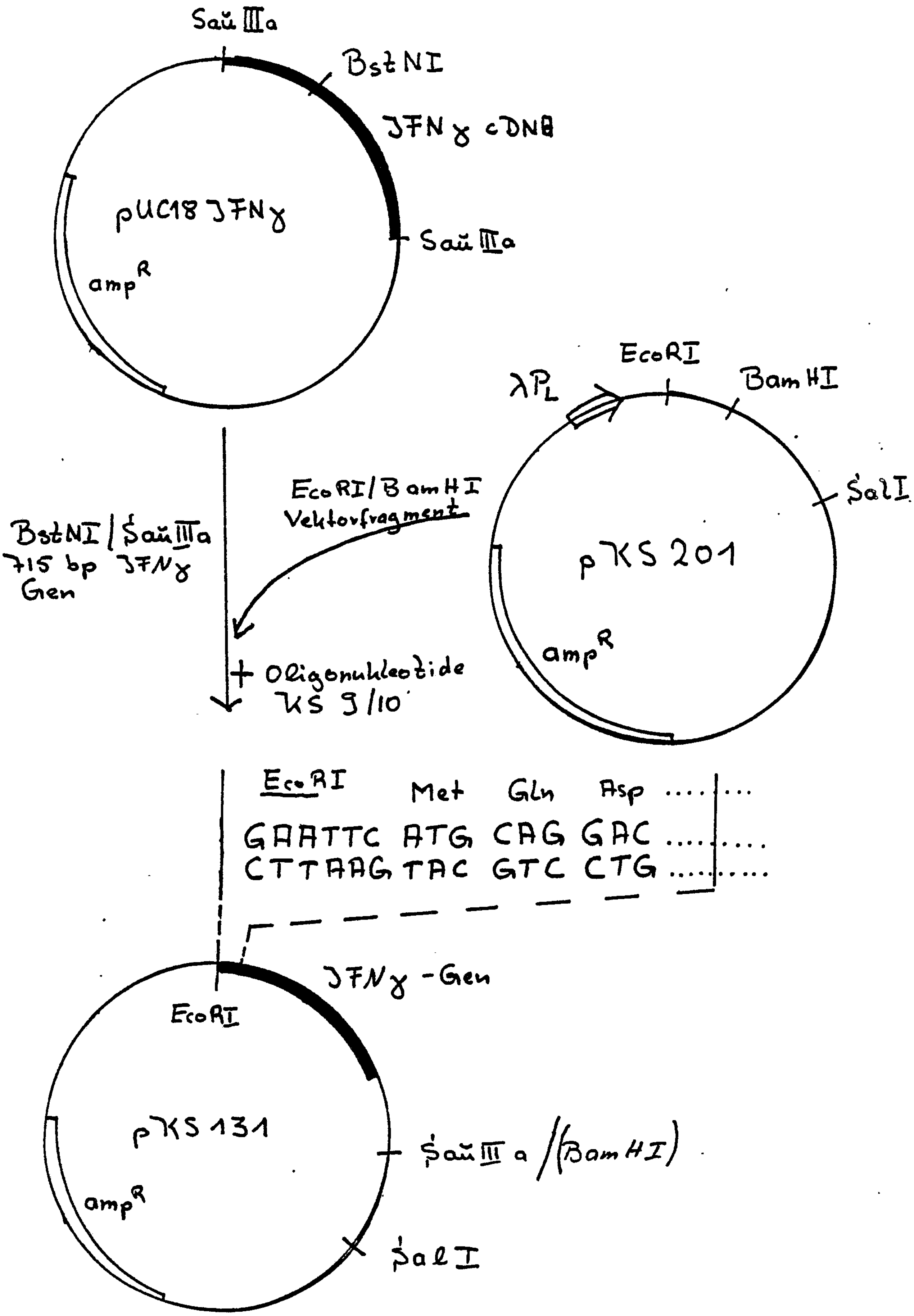

Saŭ IIIa
BstNI
JFNγ cDNA
pUC18 JFNγ
Saŭ IIIa
amp^R
EcoRI
λPL
BamHI
SalI
pKS 201
amp^R
EcoRI/BamHI Vektorfragment
BstNI / SaŭIIIa 715 bp JFNγ Gen
+ Oligonukleotide KS 9/10
EcoRI Met Gln Asp
GAATTC ATG CAG GAC
CTTAAG TAC GTC CTG
JFNγ -Gen
EcoRI
pKS 131
amp^R
SaŭIII a /(BamHI)
SalI